(19) Europäisches Patentamt
European Patent Office
Office européen des brevets

(11) EP 1 214 929 A2

(12) EUROPÄISCHE PATENTANMELDUNG

(43) Veröffentlichungstag:
**19.06.2002 Patentblatt 2002/25**

(51) Int Cl.$^7$: **A61K 7/42**, A61P 17/16

(21) Anmeldenummer: **01129341.2**

(22) Anmeldetag: **17.12.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **18.12.2000 DE 10063130**

(71) Anmelder: **Beiersdorf Aktiengesellschaft**
**20245 Hamburg (DE)**

(72) Erfinder:
- **Knüppel, Anja**
  **22527 Hamburg (DE)**
- **Grundt, Wiebke**
  **21244 Buchholz (DE)**
- **Schulz, Jens, Dr.**
  **12169 Berlin (DE)**

(54) **Verwendung von Polyurethanen zur Verbesserung der Wasserfestigkeit kosmetischer oder dermatologischer Formulierungen**

(57) Verwendung von filmbildenden oder in Wasser dispergierbaren Polyurethanen zur zur Verbesserung der Wasserfestigkeit kosmetischer oder dermatologischer Formulierungen.

EP 1 214 929 A2

## Beschreibung

Die vorliegende Erfindung betrifft kosmetische und dermatologische Formulierungen, insbesondere kosmetische und dermatologische Lichtschutzformulierungen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Man hat lange Zeit fälschlicherweise angenommen, daß die langwellige UV-A-Strahlung mit einer Wellenlänge zwischen 320 nm und 400 nm nur eine vemachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasem zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Femer können bereits sehr geringe Strahlendosen photochemische Reaktionen auslösen. Hierzu gehört insbesondere die Bildung freier Radikale, welche wiederum aufgrund ihrer hohen Reaktivität unkontrollierte Folgereaktionen auslösen können. Um solchen Reaktionen vorzubeugen, können den kosmetischen bzw. dermatologischen Formulierungen zusätzlich Antioxidantien und/oder Radikalfänger zugesetzt werden. So ist beispielsweise vorgeschlagen worden, Vitamin E, eine Substanz mit bekannter antioxidativer Wirkung in Lichtschutzformulierungen einzusetzen, allerdings bleibt hier die erzielte Wirkung weit hinter der erhofften zurück.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Homschicht zurückgehalten werden.

Ein Sonnenbad wird von den meisten Menschen als angenehm empfunden, die nachteiligen Folgen zunächst nicht beachtet. Allerdings hat sich in den letzten Jahren durchaus ein Bewußtsein über die negativen Auswirkungen einer zu intensiven Sonnenbestrahlung herausgebildet, weshalb mehr und stärker schützende Sonnenschutzmittel angewendet werden.

Da die Beiträge der verschiedenen Wellenlängebereiche des UV-Lichtes zu lichtbedingten Hautveränderungen nicht vollständig geklärt sind, geht man heute verstärkt davon aus, daß vorbeugender Schutz sowohl gegen UV-A- als auch gegen UV-B-Strahlen, beispielsweise durch Auftrag von Lichtschutzfiltersubstanzen in Form einer kosmetischen oder dermatologischen Formulierung auf die Haut, von grundsätzlicher Wichtigkeit ist. Kosmetische oder dermatologische Mittel sollen, in dünner Schicht auf die Haut aufgetragen, diese vor den negativen Auswirkungen der Sonnenstrahlung schützen.

Auch im Bereich der Sonnenschutzformulierungen spielen Emulsionen die entscheidende Rolle. Dabei werden fließfähige Emulsionen bevorzugt, da sie sich leichter auftragen lassen als zähflüssige Cremes. Die meisten Sonnenschutzmittel werden in Wassernähe oder bei sportlicher Betätigung (Schwitzen) angewendet, weshalb der Wasserfestigkeit solcher Formulierungen besondere Bedeutung beizumessen ist. Ein wasserfestes Sonnenschutzmittel schützt den Anwender nicht nur nach dem Baden, sondern bewahrt ihn auch während des Badens vor einem Sonnenbrand.

Um hohe Lichtschutzfaktoren bei gleichzeitiger sehr guter Wasserfestigkeit zu erreichen, sind W/O-Formulierungen in der Regel von Vorteil. Allerdings weisen W/O-Emulsionen häufig unbefriedigende kosmetische Eigenschaften auf: Bei der Anwendung können sie auf der Haut einen fettigen, glänzenden und zum Teil klebrigen Eindruck

hinterlassen und sich - insbesondere auf behaarter Haut - schwierig verteilen lassen.

O/W-Emulsionen hingegen wirken weniger fettend auf der Haut, sind eher mattierend und ziehen schnell in die Haut ein. Sie werden vom Verbraucher im allgemeinen als leichter und kosmetisch eleganter als W/O-Emulsionen empfunden. Da Wasser die äußere Phase ist, sind O/W-Emulsionen gewöhnlich allerdings nur bedingt wasserfest.

Neben dem Einfluß der Grundlage ist auch die Bindungsfähigkeit des UV-Filters in oder auf der Haut von großer Bedeutung für die Wasserfestigkeit der Formulierung. Es ist verständlich, daß öllösliche UV-Filter besser an die (lipophile) Oberfläche der Haut gebunden werden bzw. schwerer von dieser abwaschbar sind als wasserlösliche UV-Filter.

Aufgabe der Erfindung war es daher, auf einfache und preiswerte Weise zu Zubereitungen (insbesondere zu O/W-Formulierungen) zu gelangen, welche sich durch eine gute Wasserfestigkeit auszeichnen. Insbesondere sollten Zubereitungen gefunden werden, welche einen hohen Gehalt an wasserlöslichen UV-Filtern haben und dennoch sehr gute Wasserfestigkeit zeigen.

Die Verwendung von filmbildenden, wasserlöslichen oder in Wasser dispergierbaren Polymeren in kosmetischen oder dermatologischen Formulierungen ist an sich bekannt. So beschreibt beispielsweise die Firmenschrift TDS-248 der B. F. Goodrich Company die Verwendung von unterschiedlichen Polymertypen auf Polyacrylat- oder auf Polyurethanbasis in kosmetischen Formulierungen, beispielsweise zur Verbesserung des Hautgefühls. Diese Schrift konnte aber nicht den Weg zur vorliegenden Erfindung weisen.

Überraschend und für den Fachmann nicht vorauszusehen war, daß die Verwendung von filmbildenden, wasserlöslichen oder in Wasser dispergierbaren Polyurethanen zur Verbesserung der Wasserfestigkeit kosmetischer oder dermatologischer Formulierungen, enthaltend mindestens eine übliche UV-Filtersubstanz, den Nachteilen des Standes der Technik abhelfen würde.

Ferner war überraschend, daß die Verwendung von filmbildenden, wasserlöslichen oder in Wasser dispergierbaren Polyurethanen zur Verbesserung der Wasserfestigkeit von O/W-Formulierungen den Nachteilen des Standes der Technik abhelfen würde.

Es war auch erstaunlich, daß die Verwendung von filmbildenden, wasserlöslichen oder in Wasser dispergierbaren Polyurethanen zur Verbesserung der Wasserfestigkeit kosmetischer oder dermatologischer Formulierungen enthaltend mindestens eine übliche wasserlösliche UV-Filtersubstanz, den Nachteilen des Standes der Technik abhelfen würde.

Polyurethane sind Polymere, in deren Makromolekülen die Wiederholungseinheiten durch Urethan-Gruppierungen -NH-CO-O- verknüpft sind. Polyurethane werden im allgemeinen durch Polyaddition aus zwei- oder höherwertigen Alkoholen und Isocyanaten erhalten:

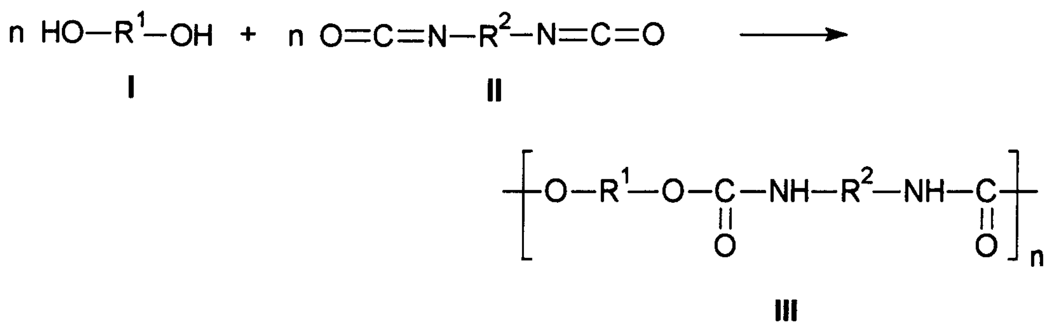

I

II

III

$R^1$ und $R^2$ können dabei für niedermolekulare oder selbst schon polymere aliphatische oder aromatische Gruppen stehen.

Vorteilhaft im Sinne der vorliegenden Erfindung sind in Wasser lösliche oder dispergierbare anionische Polyurethane *(Polyurethane A)* aus

a) mindestens einer Verbindung, die zwei oder mehrere aktive Wasserstoffatome pro Molekül enthält,
b) mindestens einem Säure- oder Salzgruppen enthaltenden Diol
und
c) mindestens einem Diisocyanat,
die eine Glastemperatur von mindestens 15°C und Säurezahlen im Bereich von 12 bis 150, vorzugsweise 30 bis 90, besitzen
und die Salze davon.

Bei der Komponente a) handelt es sich insbesondere um Diole, Aminoalkohole, Diamine, Polyesterole, Polyetherole mit einem zahlenmittleren Molekulargewicht von jeweils bis zu 3000 oder deren Mischungen, wobei bis zu 3 Mol-% der genannten Verbindungen durch Triole oder Triamine ersetzt sein können. Bevorzugt sind Diole und Polyesterdiole. Insbesondere umfaßt die Komponente (a) mindestens 50 Gew.-%, bezogen auf das Gesamtgewicht der Komponente (a), eines Polyesterdiols. Als Polyesterdiole kommen alle diejenigen in Betracht, die üblicherweise zur Herstellung von Polyurethanen eingesetzt werden, insbesondere Umsetzungsprodukte aus Phthalsäure und Diethylenglycol, Isophthalsäure und 1,4-Butandiol, Isophthalsäure/Adipinsäure und 1,6-Hexandiol sowie Adipinsäure und Ethylenglycol oder 5-$NaSO_3$-Isophthalsäure, Phthalsäure, Adipinsäure und 1,6-Hexandiol.

Erfindungsgemäß bevorzugte Diole sind z. B. Ethylenglycol, Propylenglycol, Butylenglycol, Neopentylglycol, Polyetherole, wie beispielsweise Polyethylenglycole mit Molekulargewichten bis zu 3000, Blockcopolymerisate aus Ethylenoxid und Propylenoxid mit zahlenmittleren Molekulargewichten von bis zu 3000 oder Blockcopolymerisate aus Ethylenoxid, Propylenoxid und Butylenoxid, die die Alkylenoxideinheiten statistisch verteilt oder in Form von Blöcken einpolymerisiert enthalten. Besonders bevorzugt sind Ethylenglycol, Neopentylglycol, Di-, Tri-, Tetra-, Penta und/oder Hexaethylenglyol. Diole Auch Poly($\alpha$-hydroxycarbonsäure)diole sind vorteilhaft im Sinne der vorliegenden Erfindung.

Vorteilhafte Aminoalkohole sind z. B. 2-Aminoethanol, 2-(N-Methylamino)ethanol, 3-Aminopropanol oder 4-Aminobutanol.

Vorteilhafte Diamine sind z. B. Ethylendiamin, Propylendiamin, 1,4-Diaminobutan und 1,6-Diaminohexan sowie $\alpha,\omega$-Diamine, die durch Aminierung von Polyalkylenoxiden mit Ammoniak herstellbar sind.

Bei der Komponente b) handelt es sich insbesondere um Dimethylolpropansäure oder Verbindungen der Formeln

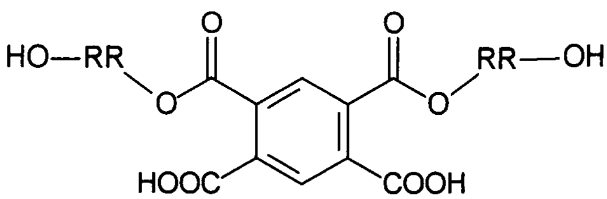

bzw.

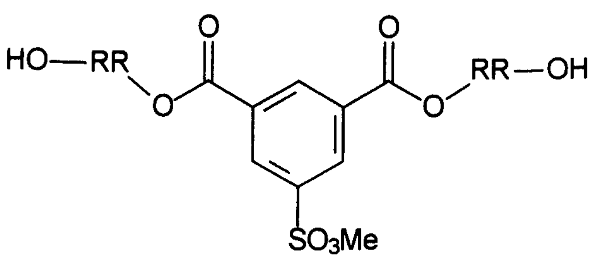

worin RR jeweils für eine $C_2$-$C_{18}$-Alkylengruppe steht und Me für Na oder K steht.

Bei der Komponente c) handelt es sich insbesondere um Hexamethylendiisocyanat, Isophorondiisocyanat, Methyldiphenylisocyanat (MDI) und/oder Toluylendiisocyanat.

Die Polyurethane sind dadurch erhältlich, daß man die Verbindungen der Gruppen a) und b) unter einer Inertgasatmosphäre in einem inerten Lösemittel bei Temperaturen von 70 bis 130°C mit den Verbindungen der Gruppe c) umsetzt. Diese Umsetzung kann gegebenenfalls in Gegenwart von Kettenverlängerern durchgeführt werden, um Polyurethane mit höheren Molekulargewichten herzustellen. Wie bei der Herstellung von Polyurethanen üblich, werden die Komponenten [(a)+(b)] : (c) vorteilhaft im molaren Verhältnis von 0,8 bis 1,1 : 1 eingesetzt. Die Säurezahl der Polyurethane wird von der Zusammensetzung und der Konzentration der Verbindungen der Komponente (b) in der Mischung aus den Komponenten (a) und (b) bestimmt.

Die Polyurethane haben K-Werte nach H. Fikentscher (bestimmt in 0,1 Gew.-%igen Lösungen in N-Methylpyrrolidon bei 25 °C und pH = 7) von 15 bis 100, vorzugsweise 25 bis 50.

Der auch als Eigenviskosität bezeichnete K-Wert ist ein über Viskositätsmessungen von Polymerlösungen

einfach zu bestimmender und daher im technischen Bereich häufig verwendeter Parameter zur Charakterisierung von Polymeren. Für eine bestimmte Polymer-Sorte wird er unter standardisierten Meßbedingungen als allein von der mittleren Molmasse der untersuchten Probe abhängig angenommen und über die Beziehung K-Wert = 1000 k nach der Fikentscher-Gleichung

$$k = \frac{1{,}5 \lg\eta_r - 1 \pm \sqrt{1 + \left(\frac{2}{c} + 2 + 1{,}5 \lg\eta_r\right) \cdot 1{,}5 \lg\eta_r}}{150 + 300c}$$

berechnet, in der bedeuten:

$\eta_r$ = relative Viskosität (dynamische Viskosität der Lösgung/dynamische Viskosität des Lösemittels) und c = Massenkonzentration an Polymer in der Lösung (in g/cm$^3$).

Die Säuregruppen enthaltenden Polyurethane sind nach Neutralisation (teilweise oder vollständig) wasserlöslich bzw. ohne Zuhilfenahme von Emulgatoren dispergierbar. In aller Regel weisen die Salze der Polyurethane eine bessere Wasserlöslichkeit oder Dispergierbarkeit in Wasser auf als die nicht neuralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Ammoniak und Amine verwendet werden. Besonders haben sich zur Neutralisation der Säuregruppen enthaltenden Polyurethane 2-Amino-2-Methylpropanol, Diethylaminopropylamin und Triisopropanolamin bewährt. Die Neutralisation der Säuregruppen enthaltenden Polyurethane kann auch mit Hilfe von Mischungen mehrerer Basen vorgenommen werden, z. B. Mischungen aus Natronlauge und Triisopropanolamin. Die Neutralisation kann je nach Anwendungszweck partiell, z. B. zu 20 bis 40 %, oder vollständig, d.h. zu 100 %, erfolgen.

Diese Polymere und ihre Herstellung sind in DE-A-42 25 045 näher beschrieben, auf die hiermit in vollem Umfang Bezug genommen wird.

Vorteilhaft im Sinne der vorliegenden Erfindung sind ferner in Wasser lösliche oder dispergierbare, kationische Polyurethane und Polyharnstoffe aus

a) mindestens einem Diisocyanat, welches bereits vorher mit einer oder mehreren Verbindungen, die zwei oder mehrere aktive Wasserstoffatome pro Moleküle enthalten, umgesetzt worden sein kann, und
b) mindestens einem Diol, primären oder sekundären Aminoalkohol, primären oder sekundärem Diamin oder primären oder sekundären Triamin mit einem oder mehreren tertiären, quaternären oder protonierten tertiären Aminostickstoffatomen,

die eine Glasübergangstemperatur von mindestens 25°C und eine Aminzahl von 50 bis 200, bezogen auf die nichtquatemisierten oder protonierten Verbindungen aufweisen, und die Salze davon. Die Aminzahl liegt vorzugsweise im Bereich von 65 bis180, insbesondere 70 bis 170, besonders bevorzugt 75 bis 160 und ganz besonderes 80 bis 150.

Bevorzugte Diisocyanate sind die für die Polyurethane A angegebenen. Verbindungen mit zwei oder mehreren aktiven Wasserstoffatomen sind Diole, Aminoalkohole, Diamine, Polyesterole. Polyamiddiamine und Polyetherole. Vorteilhafte Verbindungen dieser Art sind die für die Polyurethane A angegebenen.

Die Herstellung der Polyurethane erfolgt wie für die Polyurethane A beschrieben. Geladene kationische Gruppierungen lassen sich aus den vorliegenden tertiären Aminostickstoffatomen entweder durch Protonierung, z. B. mit Carbonsäuren wir Milchsäure, oder durch Quatemisierung, z. B. mit Alkylierungsmitteln wie $C_1$-bis $C_4$-Alkylhalogeniden oder -sulfaten in den Polyharnstoffen erzeugen. Beispiele solcher Alkylierungsmittel sind Ethylchlorid, Ethylbromid, Methylchlorid, Methylbromid, Dimethylsulfat und Diethylsulfat.

Diese Polymere und ihre Herstellung sind in der DE-A-42 41 118 näher beschrieben, auf die hiermit in vollem Umfang Bezug genommen wird.

Vorteilhaft im Sinne der vorliegenden Erfindung sind ferner lineare Polyurethane mit Carboxylatgruppen aus

a) einer 2,2-Hydroxymethyl-substituierten Carbonsäure der Formel

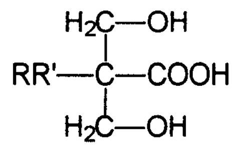

worin RR' für ein Wasserstoffatom oder eine $C_1$-$C_{20}$-Alkylgruppe steht, die in einer Menge verwendet wird, welche ausreicht, daß in dem Polyurethan 0,35 bis 2,25 Milliäquivalente Carboxylgruppen pro g Polyurethan vorhanden sind,

b) 10 bis 90 Gew.-%, bezogen auf das Gewicht des Polyurethans, einer oder mehrerer organischer Verbindungen mit nicht mehr als zwei aktiven Wasserstoffatomen und

c) einem oder mehreren organischen Diisocyanaten.

Die im Polyurethan enthaltenden Carboxylgruppen werden abschließend mit einer geeigneten Base zumindest teilweise neutralisiert. Diese Polymere und ihre Herstellung sind in der EP-A-619 111 beschrieben, auf die hiermit in vollem Umfang Bezug genommen wird.

Vorteilhaft im Sinne der vorliegenden Erfindung sind ferner carboxylhaltige Polykondensationsprodukte mit Glastemperaturen von >20°C aus Anhydriden von Trioder Tetracarbonsäuren und Diolen, Diaminen oder Aminoalkoholen (Polyester, Polyamide oder Polyesteramide). Diese Polymere und ihre Herstellung sind in der DE-A-42 24 761 näher beschrieben, auf die hiermit in vollem Umfang Bezug genommen wird.

Die erfindungsgemäß zur Anwendung kommenden Polymere besitzen vorzugsweise einen K-Wert von 25 bis 100, bevorzugt 25 bis 50.

Vorteilhaft im Sinne der vorliegenden Erfindung sind z. B. Polyurethan-1 und/oder Polyurethan-4.

Besonders vorteilhafte Polyurethane im Sinne der vorliegenden Erfindung sind die unter der Handelsbezeichnung Avalure™ UR bei der B. F. Goodrich Company erhältlichen Typen, wie beispielsweise Avalure™ UR 445, Avalure™ UR 450 und dergleichen. Ferner vorteilhaft im Sinne der vorliegenden Erfindung ist auch das unter der Handelsbezeichnung Luviset Pur bei der BASF erhältliche Polyurethan.

Kosmetische oder dermatologische Zubereitungen im Sinne der vorliegenden Erfindung enthalten - bezogen auf das Gesamtgewicht der Zubereitungen - vorteilhaft 0,1 bis 5 Gew.-%, insbesondere 0,2 bis 2,5 Gew.-% filmbildende Polyurethane.

Die erfindungsgemäßen kosmetischen und/oder dermatologischen Formulierungen können wie üblich zusammengesetzt sein und dem kosmetischen und/oder dermatologischen Lichtschutz, femer zur Behandlung, der Pflege und der Reinigung der Haut und/oder der Haare und als Schminkprodukt in der dekorativen Kosmetik dienen. Entsprechend können die erfindungsgemäßen Zubereitungen, je nach ihrem Aufbau, beispielsweise verwendet werden als Hautschutcrème, Reinigungsmilch, Sonnenschutzlotion, Nährcrème, Tages- oder Nachtcrème usw. Es ist gegebenenfalls möglich und vorteilhaft, die erfindungsgemäßen Zubereitungen als Grundlage für pharmazeutische Formulierungen zu verwenden. Bevorzugt sind insbesondere solche kosmetischen und dermatologischen Zubereitungen, die in der Form eines Hautpflege- bzw. Schminkproduktes vorliegen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise neben einer oder mehreren erfindungsgemäßen UV-Filtersubstanzen zusätzlich mindestens eine weitere UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans ($TiO_2$), Zinks (ZnO), Eisens (z. B. $Fe_2O_3$), Zirkoniums ($ZrO_2$),

Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden.

Solche Pigmente können im Sinne der vorliegenden Erfindung vorteilhaft oberflächlich behandelt ("gecoatet") sein, wobei beispielsweise ein amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Erfindungsgemäß vorteilhaft sind z. B. Titandioxidpigmente, die mit Octylsilanol beschichtet sind. Geeignete Titandioxidpartikel sind unter der Handelsbezeichnung T805 bei der Firma Degussa erhältlich. Besonders vorteilhaft sind ferner mit Aluminiumstearat beschichtete $TiO_2$-Pigmente, z. B. die unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCl: Bisoctyltriazol], welches durch die chemische Strukturformel

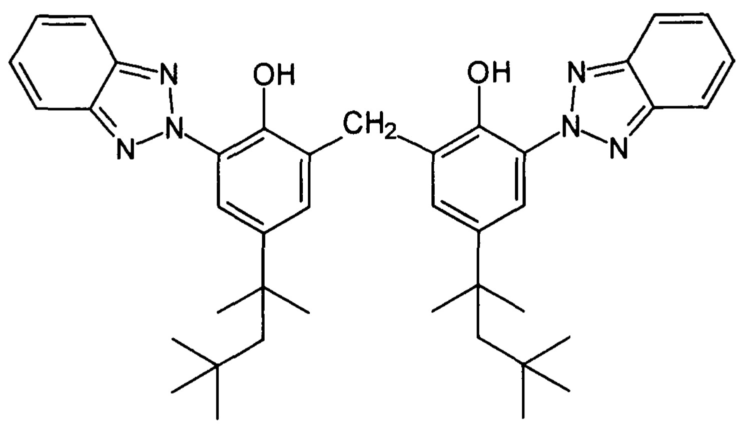

gekennzeichnet ist und unter der Handelsbezeichnung Tinosorb® M bei der CIBA-Chemikalien GmbH erhältlich ist.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure

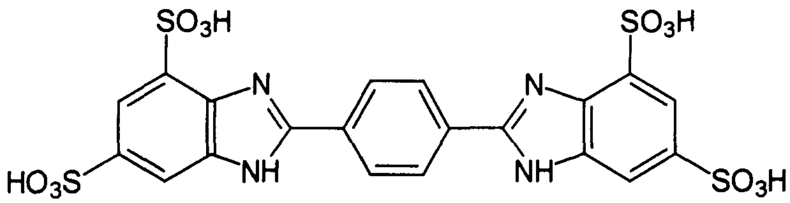

und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz

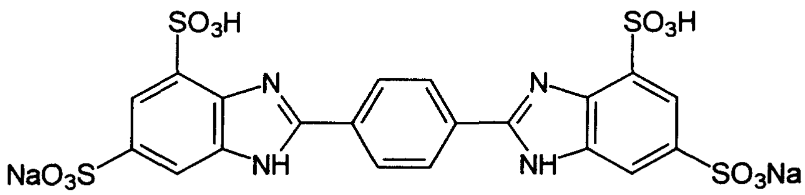

mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

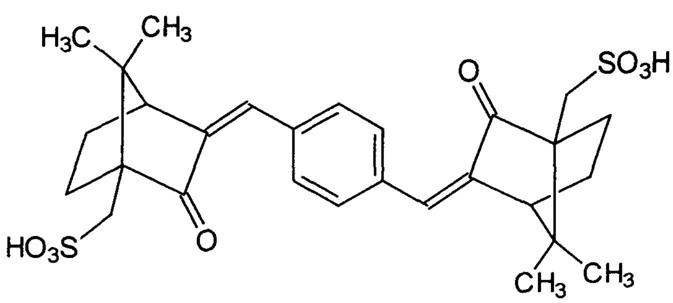

Vorteilhafte UV-Filtersubstanzen im Sinne der vorliegenden Erfindung sind femer sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur:

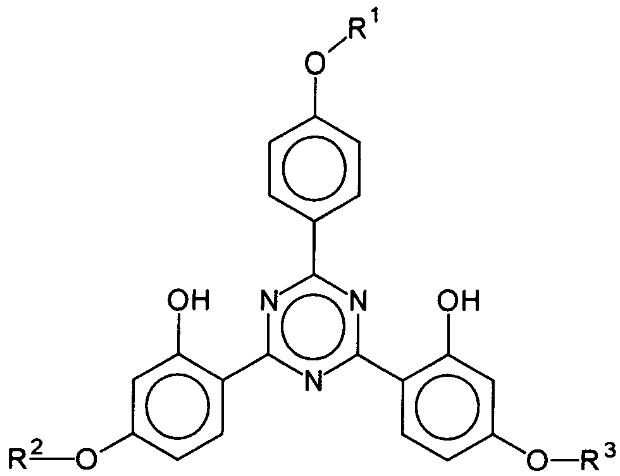

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv

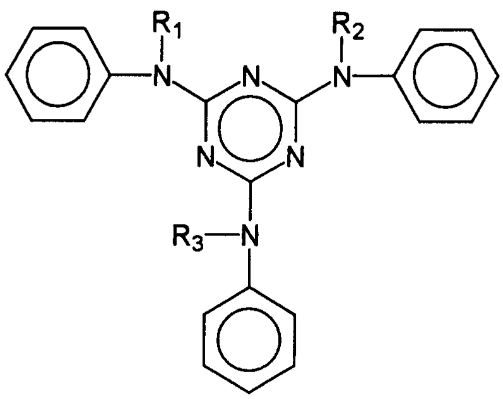

aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel

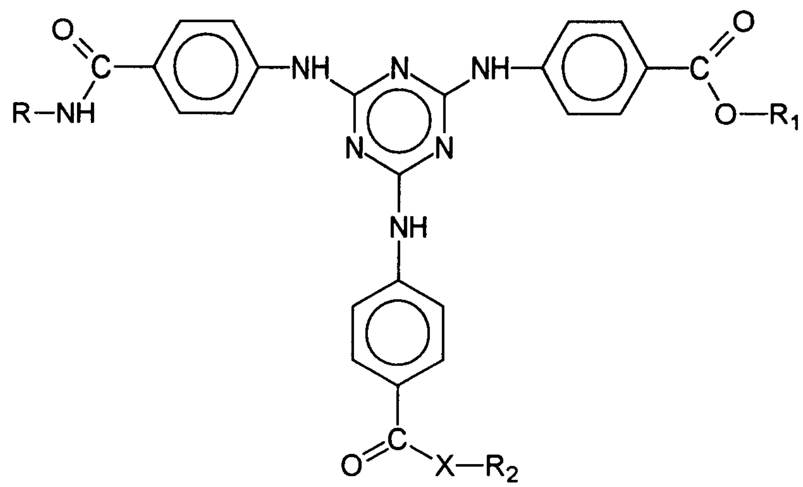

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

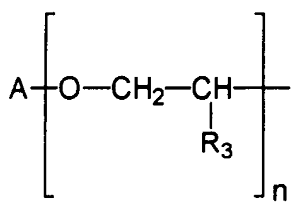

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

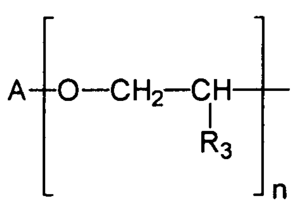

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,
$R_3$ ein Wasserstoffatom oder eine Methylgruppe darstellt,
n eine Zahl von 1 bis 10 darstellt,
wenn X ein Sauerstoffatom darstellt.

Besonders bevorzugte UV-Filtersubstanz im Sinne der vorliegenden Erfindung ist femer ein unsymmetrisch substituiertes s-Triazin, dessen chemische Struktur durch die Formel

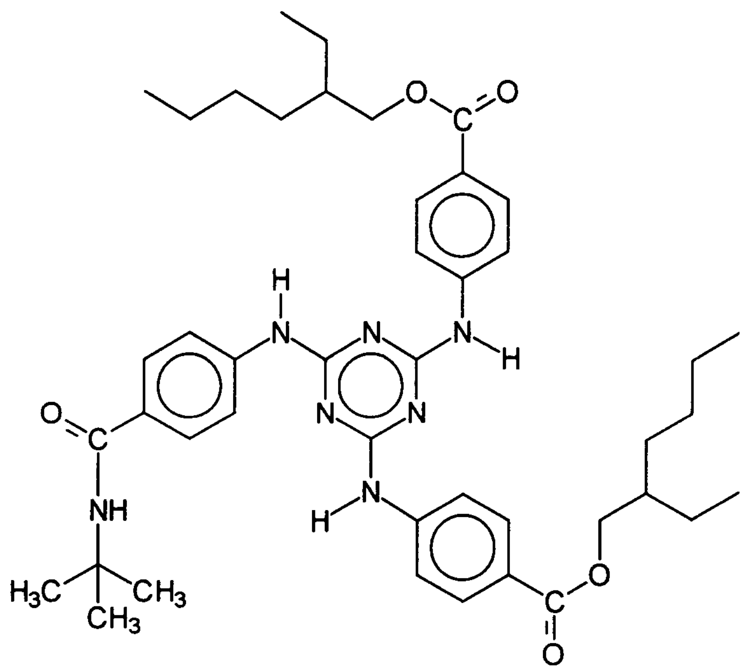

wiedergegeben wird, welches im Folgenden auch als Dioctylbutylamidotriazon (INCI: Dioctylbutamidotriazone) bezeichnet wird und unter der Handelsbezeichnung UVA-SORB HEB bei Sigma 3V erhältlich ist.

Vorteilhaft im Sinne der vorliegenden Erfindung ist auch ein symmetrisch substituiertes s-Triazin, das 4,4',4''-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel

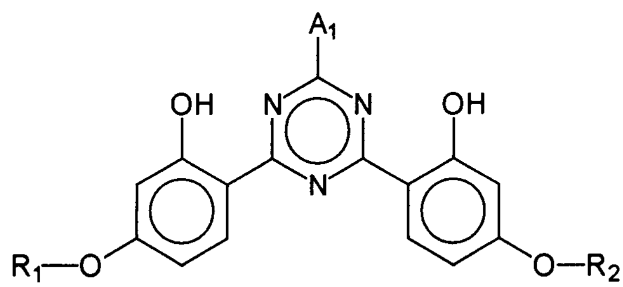

wiedergegeben wird, wobei $R_1$ , $R_2$ und $A_1$ verschiedenste organische Reste repräsentieren.

Vorteilhaft im Sinne der vorliegenden Erfindung sind femer das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2''-methylpropenyloxy)-2-hydroxy]-phenyl)-6-(4-methoxyphenyl)-1,3,5-triazin und das 2,4-Bis-{[4-(1',1',1',3',5',5',5'-Heptamethylsiloxy-2''methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel

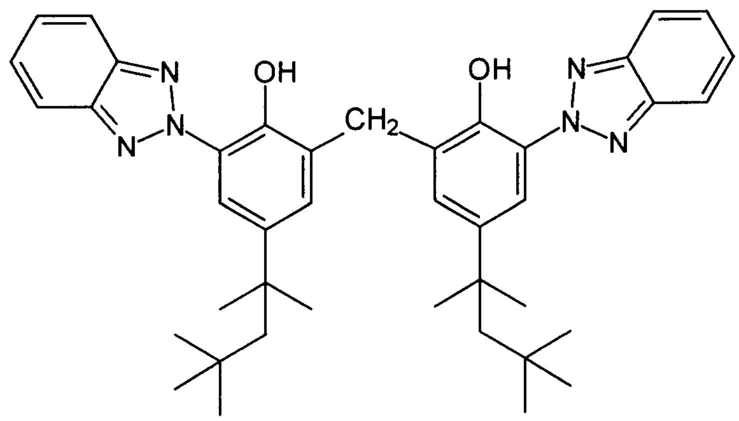

gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist femer das 2-(2Hbenzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel

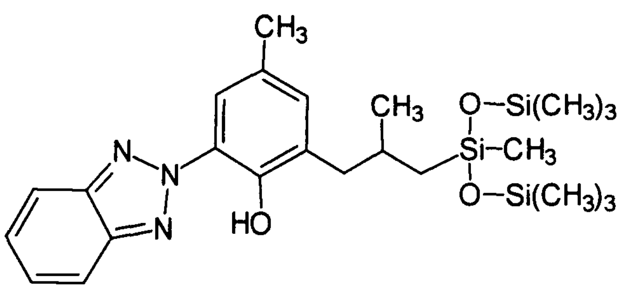

gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- sowie an Polymere gebundene UV-Filter.

Vorteilhafte wasserlösliche UV-B- und/oder Breitband-Filtersubstanzen sind z. B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z. B. 4-(2-Oxo-3-bomylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

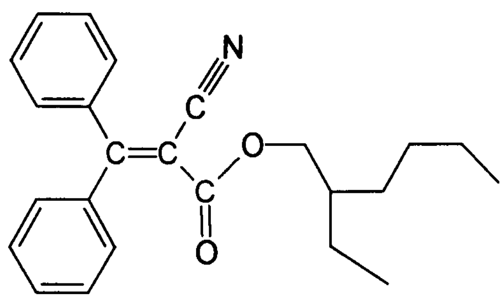

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Femer kann es gegebenenfalls von Vorteil sein, erfindungsgemäß weitere UV-Aund/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die kosmetischen und dermatologischen Zubereitungen im Sinne der vorliegenden Erfindung stellen bevorzugt disperse Zwei- oder Mehrphasensysteme dar, welche neben einer oder mehrerer Ölphasen zusätzlich eine oder mehrere Wasserphasen enthalten können. Sie können in Form kosmetischer oder dermatologischer Emulsionen - beispielsweise vom Typ W/O, O/W, W/O/W, O/W/O und dergleichen - vorliegen. Solche Emulsionen können vorzugsweise auch eine Mikroemulsion, eine Pickering-Emulsion oder eine sprühbare Emulsion sein. Besonders bevorzugt im Sinne der vorliegenden Erfindung sind allerdings Formulierungen, in denen Wasser die äußere Phase ist, insbesondere O/W-Emulsionen, Hydrodispersionen oder wäßrige Systeme, welche wasserlösliche UV-Filtersubstanzen enthalten.

Die kosmetischen und dermatologischen Zubereitungen im Sinne der vorliegenden Erfindung können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die jeweils einzusetzenden Mengen an kosmetischen oder dermatologischen Hilfs- und Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Camosin, D-Carnosin, L-Camosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis μmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, $ZnSO_4$) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Die Ölphase der Zubereitungen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether und Dicaprylylcarbonate.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase femer einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind femer Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alko hole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, femer Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispiele:**

| **1. PIT - Sonnen Sprays** | | | | | |
|---|---|---|---|---|---|
| | **1** | **2** | **3** | **4** | **5** |
| Glycerinmonostearate SE | 0,50 | | 3,00 | 2,00 | 4,00 |
| Ceteareth-12 | | 5,00 | | 1,00 | 1,50 |
| Ceteareth-20 | | | | 2,00 | |
| Ceteareth-30 | 5,00 | | 1,00 | | |
| Stearyl Alkohol | | | 3,00 | | 0,50 |
| Cetyl Alkohol | 2,50 | 1,00 | | 1,50 | |
| Ethylhexyl Methoxycinnamate | | | | 5,00 | 8,00 |
| Aniso Triazine | | 1,50 | | 2,00 | 2,50 |
| Butyl Methoxydibenzoylmethane | | | 2,00 | | |
| Dioctyl Butamidotriazon | 1,00 | 2,00 | | 2,00 | |
| Ethylhexyl Triazone | 4,00 | | 3,00 | 4,00 | |
| 4-Methylbenzylidene Camphor | | 4,00 | | | 2,00 |
| Octocrylene | | 4,00 | | | 2,50 |
| Bisimidazylate | | | 0,50 | | 1,50 |
| Phenylbenzmidazole Sulfonic Acid | 0,50 | | | 3,00 | |
| Bisoctyltriazol | | 2,50 | | 1,00 | |
| C12-15 Alkyl Benzoate | | 2,50 | | | 5,00 |
| Dicaprylyl Ether | | | 3,50 | | |
| Butylene Glycol Dicapryiate/Dicaprate | 5,00 | | | 6,00 | |
| Dicaprylyl Carbonate | | | 6,00 | | 2,00 |
| Dimethicone | | 0,50 | 1,00 | | |

(fortgesetzt)

| 1. PIT - Sonnen Sprays | | | | | |
|---|---|---|---|---|---|
| | 1 | 2 | 3 | 4 | 5 |
| Phenyltrimethicone | 2,00 | | | 0,50 | 0,50 |
| Shea Butter | | 2,00 | | | 0,50 |
| PVP Hexadecene Copolymer | 0,50 | | | 0,50 | 1,00 |
| Glycerin | 3,00 | 7,50 | 5,00 | 7,50 | 2,50 |
| Vitamin E Acetat | 0,50 | | 0,25 | | 1,00 |
| Polyurethane | 0,20 | 0,50 | 1,50 | 0,50 | 0,40 |
| DMDM Hydantoin | 0,60 | | 0,40 | 0,20 | |
| Konkaben LMB ® | | 0,20 | | | 0,15 |
| Methylparaben | | 0,50 | 0,25 | 0,15 | |
| Phenoxyethanol | 0,50 | 0,40 | | 1,00 | 0,60 |
| Ethanol | 3,00 | 2,00 | 1,50 | | 1,00 |
| Parfuem | q.s. | q.s. | q.s. | q.s. | q.s. |
| Wasser | ad. 100 | ad. 100 | ad. 100 | ad. 100 | ad. 100 |

| 2. O/W Sonnenschutz Emulsionen | | | | | | | |
|---|---|---|---|---|---|---|---|
| | 1 | 2 | 3 | 4 | 5 | 6 | 7 |
| Glycerinmonostearate SE | 0,50 | 1,00 | 3,00 | | | 1,50 | |
| Glyceryl Stearate Citrate | 2,00 | | | 1,00 | 2,00 | | 2,50 |
| Stearinsäure | | 3,00 | | 2,00 | | | |
| PEG-40 Stearate | 0,50 | | | | | 2,00 | |
| Cetyl Phosphate | | | | | 1,00 | | |
| Stearyl Alkohol | | | 3,00 | | | 2,00 | 0,50 |
| Cetyl Alkohol | 2,50 | 1,00 | | 1,50 | 0,50 | | 2,00 |
| Ethylhexyl Methoxycinnamate | | | | 5,00 | 6,00 | | 8,00 |
| Aniso Triazine | | 1,50 | | 2,00 | 2,50 | | 2,50 |
| Butyl Methoxydibenzoylmethane | 1,00 | | 2,00 | | | 2,00 | |
| Dioctyl Butamidotriazon | | 1,00 | | 3,00 | | 2,00 | |
| Ethylhexyl Triazone | 4,00 | | 3,00 | 4,00 | 4,00 | 2,00 | |
| 4-Methylbenzylidene Camphor | 4,00 | 4,00 | | | 2,00 | 4,00 | 2,00 |
| Octocrylene | | 4,00 | | | | | 2,50 |
| Bisoctyltriazol | 1,00 | | | 2,00 | 1,00 | | |
| Bisimidazylate | 1,00 | | 0,50 | | | 1,00 | 1,50 |
| Phenylbenzmidazole Sulfonic Acid | 0,50 | | | 3,00 | | | |
| Titandioxid | 1,00 | 1,50 | | 3,00 | 2,00 | 2,00 | |
| C12-15 Alkyl Benzoate | | 2,50 | | | 4,00 | 7,00 | 5,00 |
| Dicaprylyl Ether | | | 3,50 | | 2,00 | | |

(fortgesetzt)

| 2. O/W Sonnenschutz Emulsionen | | | | | | | |
|---|---|---|---|---|---|---|---|
| | 1 | 2 | 3 | 4 | 5 | 6 | 7 |
| Butylene Glycol Dicaprylate/Dicaprate | 5,00 | | | 6,00 | | | |
| Dicaprylyl Carbonate | | | 6,00 | | | 2,00 | 2,00 |
| Dimethicone | | 0,50 | 1,00 | | 2,00 | | |
| Cyclomethicone | 2,00 | | | 0,50 | | | 0,50 |
| Shea Butter | | 2,00 | | | | | 0,50 |
| PVP Hexadecene Copolymer | 0,50 | | | 0,50 | 1,00 | | 1,00 |
| Glycerin | 3,00 | 7,50 | | 7,50 | 5,00 | | 2,50 |
| Xanthan Gummi | 0,15 | | 0,05 | | | | 0,30 |
| Sodium Carbomer | | 0,20 | 0,10 | 0,20 | | | |
| Vitamin E Acetat | 0,50 | | 0,25 | | 0,75 | | 1,00 |
| Polyurethane | 0,50 | 0,20 | 1,50 | 0,50 | 0,60 | 1,00 | 0,40 |
| DMDM Hydantoin | | 0,60 | 0,40 | 0,20 | | | |
| Konkaben LMB ® | | | | 0,18 | 0,20 | 0,10 | 0,15 |
| Methylparaben | 0,15 | | 0,25 | | 0,50 | | |
| Phenoxyethanol | 1,00 | 0,40 | | 0,40 | 0,50 | 0,40 | 0,60 |
| Ethanol | | 2,00 | 1,50 | | 3,00 | | 1,00 |
| Parfuem | q.s. | q.s. | q.s. | q.s. | q.s. | q.s. | q.s. |
| Wasser | ad. 100 | ad. 100 | ad. 100 | ad. 100 | ad. 100 | ad. 100 | ad. 100 |

| 3. Hydrodispersionen | | | | | |
|---|---|---|---|---|---|
| | 1 | 2 | 3 | 4 | 5 |
| Ceteareth-20 | 1,00 | | | 0,5 | |
| Cetyl Alkohol | | | 1,00 | | |
| Sodium Carbomer | | 0,20 | | 0,30 | |
| Acrylates/C10-30 Alkyl Acrylate Crosspolymer | 0,50 | | 0,40 | 0,10 | 0,10 |
| Xanthan Gummi | | 0,30 | 0,15 | | 0,50 |
| Ethylhexyl Methoxycinnamate | | | | 5,00 | 8,00 |
| Aniso Triazine | | 1,50 | | 2,00 | 2,50 |
| Butyl Methoxydibenzoylmethane | 1,00 | | 2,00 | | |
| Dioctyl Butamidotriazon | | 2,00 | | 2,00 | 1,00 |
| Ethylhexyl Triazone | 4,00 | | 3,00 | 4,00 | |
| 4-Methylbenzylidene Camphor | 4,00 | 4,00 | | | 2,00 |
| Octocrylene | | 4,00 | 4,00 | | 2,50 |
| Bisoctyltriazol | 1,00 | | | 2,00 | |
| Bisimidazylate | 1,00 | | 0,50 | | 2,00 |

(fortgesetzt)

| 3. Hydrodispersionen | | | | | |
|---|---|---|---|---|---|
| | **1** | **2** | **3** | **4** | **5** |
| Phenylbenzmidazole Sulfonic Acid | 0,50 | | | 3,00 | |
| Titandioxid | 0,50 | | 2,00 | 3,00 | 1,00 |
| C18-36 Acid Triglyceride | | 2,00 | | 1,00 | |
| C12-15 Alkyl Benzoate | 2,00 | 2,50 | | | |
| Dicaprylyl Ether | | 4,00 | | | |
| Butylene Glycol Dicaprylate/Dicaprate | 4,00 | | 2,00 | 6,00 | |
| Dicaprylyl Carbonate | | 2,00 | 6,00 | | |
| Dimethicone | | 0,50 | 1,00 | | |
| Phenyltrimethicone | 2,00 | | | 0,50 | 2,00 |
| Shea Butter | | 2,00 | | | |
| PVP Hexadecene Copolymer | 0,50 | | | 0,50 | 1,00 |
| Octoxyglycerin | | | 1,00 | | 0,50 |
| Glycerin | 3,00 | 7,50 | | 7,50 | 2,50 |
| Glycine Soja | | | 1,50 | | |
| Vitamin E Acetat | 0,50 | | 0,25 | | 1,00 |
| Polyurethane | 0,15 | 0,60 | 1,50 | 1,00 | 0,80 |
| DMDM Hydantoin | | 0,60 | 0,40 | 0,20 | |
| Konkaben LMB ® | 0,20 | | | | 0,15 |
| Methylparaben | 0,50 | | 0,25 | 0,15 | |
| Phenoxyethanol | 0,50 | 0,40 | | 1,00 | 0,60 |
| Ethanol | 3,00 | 2,00 | 1,50 | | 1,00 |
| Parfuem | q.s. | q.s. | q.s. | q.s. | q.s. |
| Wasser | ad. 100 | ad. 100 | ad. 100 | ad. 100 | ad. 100 |

| 4. W/O Sonnenschutz Emulsionen | | | | | |
|---|---|---|---|---|---|
| | **1** | **2** | **3** | **4** | **5** |
| Cetyldimethicone Copolyol | | 2,50 | | 4,00 | |
| Polyglyceryl-2-dipolyhydroxystearat | 5,00 | | | | 4,50 |
| PEG-30-dipolyhydroxystearat | | | 5,00 | | |
| Ethylhexyl Methoxycinnamate | | 8,00 | | 5,00 | 4,00 |
| Aniso Triazine | 2,00 | 2,50 | | 2,00 | 2,50 |
| Butyl Methoxydibenzoylmethane | | | 2,00 | 1,00 | |
| Dioctyl Butamidotriazon | 3,00 | 1,00 | | | 3,00 |
| Ethylhexyl Triazone | | | 3,00 | 4,00 | |
| 4-Methylbenzylidene Camphor | | 2,00 | | 4,00 | 2,00 |
| Octocrylene | 7,00 | 2,50 | 4,00 | | 2,50 |

(fortgesetzt)

| 4. W/O Sonnenschutz Emulsionen | | | | | |
|---|---|---|---|---|---|
| | 1 | 2 | 3 | 4 | 5 |
| Bisoctyltriazol | 1,00 | | | 2,00 | |
| Bisimidazylate | 1,00 | 2,00 | 0,50 | | |
| Phenylbenzmidazole Sulfonic Acid | 0,50 | | | 3,00 | 2,00 |
| Titandioxid | | 2,00 | 1,50 | | 3,00 |
| Mineralöl | | | 10,0 | | 8,00 |
| C12-15 Alkyl Benzoate | | | | 9,00 | |
| Dicaprylyl Ether | 10,00 | | | | 7,00 |
| Butylene Glycol Dicaprylate/Dicaprate | | | 2,00 | 8,00 | 4,00 |
| Dicaprylyl Carbonate | 5,00 | | 6,00 | | |
| Dimethicone | | 4,00 | 1,00 | 5,00 | |
| Cyclomethicone | 2,00 | 25,00 | | | 2,00 |
| Shea Butter | | | 3,00 | | |
| PVP Hexadecene Copolymer | 0,50 | | | 0,50 | 1,00 |
| Octoxyglycerin | | 0,30 | 1,00 | | 0,50 |
| Glycerin | 3,00 | 7,50 | | 7,50 | 2,50 |
| Glycine Soja | | 1,00 | 1,50 | | |
| $MgSO_4$ | 1,00 | 0,50 | | 0,50 | |
| $MgCl_2$ | | | 1,00 | | 0,70 |
| Vitamin E Acetat | 0,50 | | 0,25 | | 1,00 |
| Polyurethane | 0,10 | 0,60 | 1,50 | 1,00 | 0,80 |
| DMDM Hydantoin | | 0,60 | 0,40 | 0,20 | |
| Methylparaben | 0,50 | | 0,25 | 0,15 | |
| Phenoxyethanol | 0,50 | 0,40 | | 1,00 | 0,60 |
| Ethanol | 3,00 | | 1,50 | | 1,00 |
| Parfuem | q.s. | q.s. | q.s. | q.s. | q.s. |
| Wasser | ad.100 | ad. 100 | ad. 100 | ad. 100 | ad. 100 |

## Patentansprüche

1. Verwendung von filmbildenden, wasserlöslichen oder in Wasser dispergierbaren Polyurethanen zur Verbesserung der Wasserfestigkeit kosmetischer oder dermatologischer Formulierungen, enthaltend mindestens eine übliche UV-Filtersubstanz.

2. Verwendung von filmbildenden, wasserlöslichen oder in Wasser dispergierbaren Polyurethanen zur Verbesserung der Wasserfestigkeit von O/W-Formulierungen.

3. Verwendung nach Anspruch 2, **dadurch gekennzeichnet, daß** die O/W-Formulierung eine Emulsion ist.

4. Verwendung nach Anspruch 2, **dadurch gekennzeichnet, daß** die O/W-Formulierung eine Mikroemulsion ist.

5. Verwendung nach Anspruch 2, **dadurch gekennzeichnet, daß** die O/W-Formulierung eine Hydrodispersion ist.

**6.** Verwendung von filmbildenden, wasserlöslichen oder in Wasser dispergierbaren Polyurethanen zur Verbesserung der Wasserfestigkeit kosmetischer oder dermatologischer Formulierungen enthaltend mindestens eine übliche wasserlösliche UV-Filtersubstanz.

**7.** Verwendung nach einem der vorhergehenden Ansprüche, **dadurch gekennzeichnet, daß** die kosmetischen oder dermatologischen Zubereitungen - bezogen auf ihr Gesamtgewicht - 0,1 bis 10 Gew.-% an Polyurethanen enthalten.

**8.** Verwendung nach einem der vorhergehenden Ansprüche, **dadurch gekennzeichnet, daß** die kosmetischen oder dermatologischen Lichtschutzzubereitungen als UV-A-Filtersubstanz ein oder mehrere wasserlösliche UV-A-Filtersubstanzen, besonders wasserlösliche UV-A-Filtersubstanzen gewählt aus der Gruppe Phenylen-1,4-bis-(2-benzimidazyl)-3,3'-5,5'-tetrasulfonsäure und/oder 1,4-di(2-oxo-10-Sulfo-3-bomylidenmethyl)-Benzol und/oder ihre Salze, besonders die entsprechenden Natrium-, Kalium- oder Triethanolammonium-Salze und/oder die entprechenden 10-Sulfato-verbindungen, insbesondere das Phenylen-1,4-bis-(2-benzimidazyl)-3,3'-5,5'-tetrasulfonsäure-bis-natriumsalz, enthalten.

**9.** Verwendung nach einem der vorhergehenden Ansprüche, **dadurch gekennzeichnet, daß** mindestens ein Breitbandfilter aus der Gruppe der Bis-Resorcinyltriazinderivate gewählt wird, insbesondere das 2,4-Bis-{[4-(2-Ethylhexyloxy)-2-hydroxy]-phenyl}-6-(4-methoxyphenyl)-1,3,5-triazin.

**10.** Verwendung nach einem der vorhergehenden Ansprüche, **dadurch gekennzeichnet, daß** mindestens ein Breitbandfilter aus der Gruppe der Benzotriazolderivate gewählt wird, insbesondere das 2,2'-Methylen-bis-(6-(2H-benzotriazol-2-yl)-4-(1,1,3,3-tetramethylbutyl)-phenol) und/oder das 2-(2H-benzotriazol-2-yl)-4-methyl-6-[2-methyl-3-[1,3,3,3-tetramethyl-1-[(trimethylsilyl)oxy]disiloxanyl]propyl]-phenol.